(19) Europäisches Patentamt
European Patent Office
Office européen des brevets

(11) Veröffentlichungsnummer: **0 254 222 B1**

(12) **EUROPÄISCHE PATENTSCHRIFT**

(45) Veröffentlichungstag der Patentschrift: **26.08.92**

(51) Int. Cl.[5]: **C07D 215/28**, C07D 215/26, C07D 215/60, C07D 405/12, C07D 409/12, C07D 411/12, A01N 25/32, A01N 43/42

(21) Anmeldenummer: **87110363.6**

(22) Anmeldetag: **17.07.87**

Die Akte enthält technische Angaben, die nach dem Eingang der Anmeldung eingereicht wurden und die nicht in dieser Patentschrift enthalten sind.

# (54) Neue Chinolinoxy-Verbindungen, Verfahren zu ihrer Herstellung und ihre Verwendung als Antidots.

(30) Priorität: **23.07.86 DE 3624859**

(43) Veröffentlichungstag der Anmeldung: **27.01.88 Patentblatt 88/04**

(45) Bekanntmachung des Hinweises auf die Patenterteilung: **26.08.92 Patentblatt 92/35**

(84) Benannte Vertragsstaaten: **AT BE CH DE ES FR GB IT LI NL SE**

(56) Entgegenhaltungen:
**EP-A- 0 094 349**
**EP-A- 0 138 773**
**EP-A- 0 152 006**
**EP-A- 0 154 153**

(73) Patentinhaber: **HOECHST AKTIENGESELLSCHAFT**
**Postfach 80 03 20**
**W-6230 Frankfurt am Main 80(DE)**

(72) Erfinder: **Sohn, Erich, Dr.**
**Lerchenbergstrasse 46/1**
**W-7300 Esslingen(DE)**
Erfinder: **Handte, Reinhard, Dr.**
**Theilweg 23**
**W-8901 Gablingen(DE)**
Erfinder: **Mildenberger, Hilmar, Dr.**
**Fasanenstrasse 24**
**W-6233 Kelkheim (Taunus)(DE)**
Erfinder: **Bauer, Klaus, Dr.**
**Doorner Strasse 53 D**
**W-6450 Hanau(DE)**
Erfinder: **Bieringer, Hermann, Dr.**
**Eichenweg 26**
**W-6239 Eppstein/Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft die neuen Chinolinoxy-Verbindungen zum Schützen von Kulturpflanzen gegen schädigende Nebenwirkungen von Agrochemikalien.

Bei der Anwendung von Pflanzenbehandlungsmitteln, insbesondere bei der Anwendung von Herbiziden, können unerwünschte Schäden bei den behandelten Kulturpflanzen auftreten. Besonders bei der Applikation von Herbiziden nach dem Auflaufen der Kulturpflanzen besteht daher oft das Bedürfnis, das Risiko einer möglichen Phytotoxizität durch Zugabe sogenannter "Antidots" oder "Safener" zu vermeiden. Als solche Antidots wurde bereits eine Anzahl von Chinolinoxy-Verbindungen beschrieben (EP-A 94349, EP-A 152006).

Aus der EP-A-0138773 sind cyclische Acetale von 8-Chinolinyloxy-acetaldehyd und deren Verwendung als Safener bekannt.

Überraschenderweise wurde nun gefunden, daß die neuen Chinolinoxy-aldehyd-Derivate der Formel I in verschiedenen Kulturen gegenüber dem Stand der Technik überlegene pflanzenschützende Eigenschaften aufweisen.

Gegenstand der vorliegenden Erfindung sind daher Verbindungen der Formel

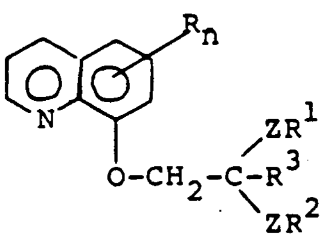

I

Verfahren zu ihrer Herstellung und ihre Verwendung als Safener für Herbizide, wobei die einzelnen Reste folgende Bedeutung haben:

R unabhängig voneinander Halogen oder $(C_1\text{-}C_4)$Alkyl,
Z unabhängig voneinander O oder S,
$R^1$, $R^2$ unabhängig voneinander H, $(C_1\text{-}C_8)$Alkyl, $(C_3\text{-}C_8)$Alkenyl, $(C_1\text{-}C_4)$Alkoxy-$(C_1\text{-}C_4)$alkyl, $(C_1\text{-}C_{12})$-Halogenalkyl mit bis zu 17 Halogenatomen, $(C_3\text{-}C_{12})$Halogenalkenyl mit bis zu 17 Halogenatomen oder Phenyl-$(C_1\text{-}C_2)$alkyl,
$R^3$ H oder $(C_1\text{-}C_4)$Alkyl und
n 0, 1, 2 oder 3

sowie deren Salze und N-Oxide.

Die Verbindungen sind - mit einer Ausnahme - neu. Bekannt ist lediglich das 8-Chinolyloxy-acetaldehyd-diethylacetal (Tokyo Gakugei Daigaku Kiyo, Dai-4-bumon 34 (1982), 55-59).

In der Definition von R bedeutet "Halogen" bevorzugt Chlor oder Brom, während in $R^1$ und $R^2$ "Halogen" vorzugsweise für Fluor oder Chlor steht.

R = $(C_1\text{-}C_4)$Alkyl bedeutet vorzugsweise Methyl.

$R^1$ und $R^2$ können z. B. folgende Bedeutungen annehmen:
Methyl, Ethyl, n-Propyl, n-Butyl, i-Butyl, i-Propyl, n-Amyl, n-Hexyl, n-Heptyl, n-Octyl, Allyl, Methallyl, Crotyl, Octenyl-(6), $CF_3$, $CCl_3$, $CCl_2CH_3$, $CHCl\text{-}CCl_3$, $CHF\text{-}CCl_3$, $CHCl\text{-}CCl_2\text{-}CH_3$, $CCl_2\text{-}CH{=}CF_2$, Benzyl oder Phenethyl.

Besonders bevorzugt sind Verbindungen der Formel I, worin R Halogen, Z Sauerstoff, $R^1$ und $R^2$ $(C_1\text{-}C_4)$Alkyl oder $(C_1\text{-}C_2)$Alkoxy-$(C_1\text{-}C_2)$alkyl, $R^3$ Wasserstoff und n 1 oder 2 bedeuten.

Unter organischen und anorganischen Säuren sind solche zu verstehen, die in der Lage sind, die Verbindungen der Formel I zu protonieren wie zum Beispiel Salzsäure, Schwefelsäure, Phosphorsäure, Salpetersäure, Oxalsäure, Trichloressigsäure, Alkyl- und Arylsulfonsäuren.

Gegenstand der Anmeldung ist weiterhin ein Verfahren zur Herstellung der Verbindungen der Formel (I), dadurch gekennzeichnet, daß man Verbindungen der Formel II

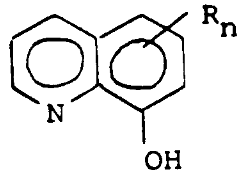

(II)

mit Verbindungen der Formel (III)

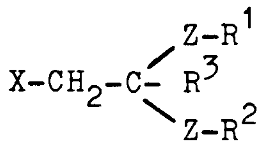

(III),

worin die Reste die oben angegebene Bedeutung haben und X für eine Abgangsgruppe steht, umsetzt und gewünschtenfalls die so erhaltene Acetale der Formel (I) durch Umacetalisierung in andere Derivate der Formel (I) bzw. Verbindungen der Formel I in ihre Salze oder N-Oxide überführt.

Die Umsetzung der Verbindungen der Formel (II) mit Verbindungen der Formel (III) erfolgt üblicherweise bei Temperaturen zwischen O° und 150°C, bevorzugt zwischen 50° und 100°C, vorteilhaft in Gegenwart eines Lösungsmittels und einr Hilfsbase. Als Lösungsmittel eignen sich z.B niedere Alkohole, Benzol, Toluol oder Dioxan; geeignete Hilfsbasen sind $K_2CO_3$ oder $Na_2CO_3$. Für einige Derivate der Formel (I) ist es vorteilhaft, die Verbindungen der Formel (II) mit einer einfach zugänglichen Verbindung der Formel (III) zu alkylieren und diese dann durch Umacetalisieren in die gewünschte Verbindung der Formel (I) zu überführen, zum Beispiel durch Erhitzen mit einem geeigneten Alkohol, Dialkohol oder Mercaptan im Überschuß oder unter Verwendung eines inerten Lösungsmittels, vorteilhaft in Gegenwart eines sauren Katalysators wie $H_2SO_4$, p-Toluolsulfonsäure oder Trifluormethansulfonsäure in einem organischen Lösemittel. Gewünschtenfalls können die Verbindung der Formel (I) nach den üblichen Methoden in ihre N-Oxide oder in ihre Salze überführt werden (vgl. J. Org. Schem. 18, 534 (1953)).

Die Verbindungen der allgemeinen Formel I zeichnen sich dadurch aus, daß sie in niedrigen, d.h. subtoxischen Konzentrationen in Kombination mit Herbiziden ausgebracht werden und dann in der Lage sind, schädliche Nebenwirkungen der letzteren zu antagonisieren, d.h. völlig aufzuheben, ohne deren herbizide Wirksamkeit zu beeinträchtigen. Hierdurch kann das Einsatzgebiet herkömmlicher Pflanzenschützmittel ganz erheblich vergrößert werden. Gegenstand der Erfindung ist daher auch ein Verfahren zum Schutz von Kulturpflanzen gegen phytotoxische Nebenwirkungen von Pflanzenschutzmitteln, insbesondere Herbiziden, das dadurch gekennzeichnet ist, daß man die Pflanzen, Pflanzenteile oder Pflanzennährböden mit einer Verbindung der Formel I vor, nach oder gleichzeitig mit de Pflanzenschutzmitteln behandelt.

Herbizide, deren phytotoxische Nebenwirkungen mittels Verbindungen der Formel I herabgesetzt werden können, sind z.B. Carbamate, Thiocarbamate, Halogenacetanilide, substituierte Phenoxy-, Napththoxy- und Phenoxyphenoxycarbonsäurederivate sowie Heteroaryloxyphenoxycarbonsäurederivate wie Chinolyloxy-, Chinoxalyloxy-, Pyridyloxy-, Benzoxazolyloxy-, Benzthiazolyloxy-phenoxycarbonsäureester und ferner Dimedonoximabkömmlinge. Bevorzugt hierin sind Phenoxy- und Heteroaryloxyphenoxy-carbonsäureester.

Beispielsweise seien, ohne daß dadurch eine Beschränkung erfolgen soll, Herbizide aus folgenden Klassen genannt:

A) Herbizide vom Typ der Phenoxyphenoxy- und Heteroaryloxyphenoxycarbonsäure-($C_1$-$C_4$)-alkyl-, subst.-($C_1$-$C_4$)-alkyl-, ($C_2$-$C_4$)alkenyl- und ($C_3$-$C_4$)-alkinylester wie

1. 2-(4-(2,4-Dichlorphenoxy)-phenoxy)-propionsäuremethylester(Diclofop-methyl),
2. 2-(4-(4-Brom-2-chlorphenoxy)-phenoxy)-propionsäuremethylester,
3. 2-(4-(4-Trifluormethylphenoxy)-phenoxy)-propionsäuremethylester,
4. 2-(4-(2-Chlor-4-trifluormethylphenoxy)-phenoxy)-propionsäuremethylester,
5. 2-(4-(2,4-Dichlorbenzyl)-phenoxy-propionsäuremethylester,
6. 4-(4-(4-Trifluormethylphenoxy)-phenoxy)-pent-2-en-säureethylester,
7. 2-(4-(3,5-Dichlorpyridyl-2-oxy)-phenoxy)-propionsäureethylester,
8. 2-(4-(3,5-Dichlorpyridyl-2-oxy)-phenoxy)-propionsäurepropargylester,
9. 2-(4-(6-Chlorbenzoxazol-2-yl-oxy)-phenoxy)-propionsäureethylester(Fenoxaprop-ethyl),

10. 2-(4-(6-Chlorbenzthiazol-2-yloxy)-phenoxy)-propionsäureethylester,
11. 2-(4-(3-Chlor-5-trifluormethyl-2-pyridyloxy)-phenoxy)-propionsäuremethylester,
12. 2-(4-(5-Trifluormethyl-2-pyridyloxy)-phenoxy)-propionsäurebutylester(Fluazifop-butyl),
13. 2-(4-(6-Chlor-2-chinoxalinyloxy)-phenoxy)-propionsäureethylester
14. 2-(4-(3,5-Dichlorpyridyl-2-oxy)-phenoxy)-propionsäurethimethylsilylmethylester
15. 2-(4-(3-Chlor-5-trifluormethoxy-2-pyridyloxy)-phenoxy)-propionsäureethylester,

B) Chloracetanilide wie
1. N-Methoxymethyl-2,6-diethyl-chloracetanilid,
2. N-(3′-Methoxyprop-2′-yl)-2-methyl-6-ethyl-chloracetanilid,
3. N-(3-Methyl-1,2,4-oxdiazol-5-ylmethyl)-chloressigsäure-2,6-dimethylanilid,

C) Thiocarbamate wie
1. S-Ethyl-N,N-dipropylthiocarbamat oder
2. S-Ethyl-N,N-diisobutylthiocarbamat,

D) Dimedon-Derivate wie
1. 2-(N-Ethoxybutyrimidoyl)-5-(2-ethylthiopropyl)-3-hydroxy-2-cyclohexene-1-on,
2. 2-(N-Ethoxybutyrimidoyl)-5-(2-phenylthiopropyl)-3-hydroxy-2-cyclohexen-1-on oder
3. 2-(1-Allyloxyiminobutyl)-4-methoxycarbonyl-5,5-dimethyl-3-oxocyclohexenol
4. 2-(N-Ethoxypropionamidoyl)-5-mesityl-3-hydroxy-2-cyclohexen-1-on
5. 2-(N-Ethoxybutyrimidoyl)-3-hydroxy-5-(thian-3-yl)-2-cyclohexen-1-on,

Dabei sind sowohl die Enantiomerengemische in beliebigen Verhältnissen als auch die reinen Antipoden erfaßt.

Das Mengenverhältnis Antidot: Herbizid kann innerhalb weiter Grenzen zwischen 0,01 und 10 Teilen Antidot auf 1 Teil Herbizid schwanken. Die jeweils optimalen Mengen an Herbizid und Antidot sind abhängig vom Typ des verwendeten Herbizids bzw. Antidots sowie von der Art des zu behandelnden Pflanzenbestandes und lassen sich von Fall zu Fall durch entsprechende Versuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle, Zuckerrüben, Zuckerrohr und Sojabohne.

Die Safener können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein.

Grundsätzlich kann das Antidot vor, nach oder gleichzeitig mit einem Herbizid angewendete werden, bevorzugt ist jedoch die gleichzeitig Anwendung in Form von Tankmischungen oder gegebenenfalls Fertigformulierungen.

Für die Anwendung können die Verbindungen der Formel I mit üblichen Formulierungshilfsmitteln zu Stäubemitteln, Spritzpulvern, Dispersionen, Emulsionskonzentraten, Granulaten oder Mikrogranulaten zubereitet weden, die den Wirkstoff in Konzentrationen von 2 - 80 % enthalten und entweder als solche angewendet werden (Stäubemittel, Pellets) oder vor der Anwendung in einem Lösungmittel (Wasser) gelöst oder dispergiert werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxyethylierte Fettalkohole, Alkyl- oder Alkyphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium, oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel , z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendete werden: Alkylarylsulfonsäure Calciumsalze wie Ca-dodecylbenzolsulfonat oder nichtionische Emulgatoren wie Fettsäurepolyglykolester, Alkylarylpolyglykolether, Fettalkoholpolyglykolether, Propylenoxid- Ethylenoxid-Kondensationsprodukte, Fettalkohol-Propylenoxid-Ethylenoxid-Kondensationsprodukte, Alkylpolyglykolether, Sorbitanfettsäureester, Polyoxyethylensorbitanfettsäureester oder Polyoxethylensorbitester.

Stäubemittel kann man durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes

Inertmaterial hergestellt werden oder durch Aufbringen von Wirkstoffkonzentraten mittels Bindemitteln, z.B. Polyvinylalkohol, polyacrylsäurem Natrium oder auch Minerlölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

A. Formulierungsbeispiele

a) Ein Stäubemittel wird erhalten, indem man 10 Gew.-Teile Safener und 90 Gewichtsteile Talkum oder Inertstoff mischt und in einer Schlagmühle zerkleinert.

b) Ein in Wasser leicht dispergierbares, benetzbares Pulver wird erhalten, indem man 25 Gewichtsteile Safener, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile Safener mit 6 Gewichtsteilen Alkylphenolpolyglykolether (®Triton X 207), 3 Gewichtsteilen Isotridecanolpolyglykolether (8 Aeo) und 71 Gewichtsteilen parafinischem Mineralöl (Siedebereich z.B. ca. 255 bis über 377°C) mischt und in einer Reibkugelmühle auf eine Feinheit von unter 5 Mikron vermahlt.

d) Ein emulgierbares Konzentrat wird erhalten aus 15 Gewichtsteilen Safener, 75 Gewichtsteilen Cyclohexanon als Lösungsmittel und 10 Gewichtsteilen oxethyliertes Nonylphenol als Emulgator.

B. Herstellungsbeispiele:

Beispiel 1:

5-Chlor-8-chinolyloxy-acetdiethylacetal (1)

180 g 5-Chlor-8-hydroxychinolin, 237 g 2-Bromacetdiethylacetal und 200 g Kaliumcarbonat werden in 800 ml Dimethylformamid 4 h bei 120°C gerührt. Nach Abkühlen gießt man auf 2,5 l Wasser, saugt ab und trocknet im Vakuum, Fp 51 - 53°C.

Beispiel 2:

5-Chlor-8-chinolyloxy-acetdi(2,2,3,4,4,4-hexafluorbutan-1-yl)acetal (2)

30 g (1) werden in 100 ml 2,2,3,4,4,4-Hexafluorbutan-1-ol gelöst, mit 10 ml Schwefelsäure versetzt und 6 h bei 60°C gerührt. Dann wird das Lösungsmittel abdestilliert mit 50 ml Wasser verdünnt, mit Essigester aufgenommen, mit $NaHCO_3$-Lösung neutralgewaschen, eingeengt und über Kieselgel chromatographiert. Die Verbindung wurde durch H-NMR-Spektrum charakterisiert.

In der nachfolgenden Tabelle 1 sind beispielhaft eine Reihe von Verbindungen der allgemeinen Formel I aufgeführt.

Tabelle 1

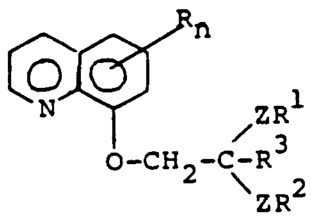

I

| Beisp.-Nr. | $R_n$ | Z | $R_1$ | $R_2$ | $R_3$ | Fp.[°C] |
|---|---|---|---|---|---|---|
| 3. | H | O | $C_2H_5$ | $C_2H_5$ | H | |
| 4. | " | " | $C_2H_4OCH_3$ | $C_2H_4OCH_3$ | H | |
| 5. | 5-Cl | " | $CH_3$ | $CH_3$ | H | 86-90 |

Tabelle 1 (Fortsetzung)

| Beisp.-Nr. | $R_n$ | Z | $R_1$ | $R_2$ | $R_3$ | Fp.[°C] |
|---|---|---|---|---|---|---|
| 6 . | " | " | $n-C_4H_9$ | $n-C_4H_9$ | H | |
| 7 . | " | " | $n-C_8H_{17}$ | $n-C_8H_{17}$ | H | Oel |
| | " | " | | | | |
| 8 . | " | " | $n-C_4H_9$ | $n-C_4H_9$ | $CH_3$ | |
| 9 . | " | " | $CH_2-Ph$ | $CH_2Ph$ | H | 109-110 |
| 10 . | " | " | $CH_2CH=CH_2$ | $CH_2CH=CH_2$ | H | |
| 11 . | " | " | $C_2H_4OCH_3$ | $C_2H_4OCH_3$ | H | Oel |
| 12 . | " | " | $C_2H_4OCH_3$ | $C_2H_5$ | H | " |
| 13 . | " | " | $CH_2CF_2CHFCF_3$ | $C_2H_5$ | H | 34-39 |
| 14 | " | " | $CH_2CH=CHC_8F_{17}(n)$ | $C_2H_5$ | H | Oel |
| 15 . | " | " | $CH_2CCl_3$ | $CH_2CCl_3$ | H | |
| 16 . | " | " | $CH_2CH_2CH_2CH_2Cl$ | $CH_2CH_2CH_2CH_2Cl$ | H | Oel |
| 17 . | " | S | $CH_2Phenyl$ | $CH_2Phenyl$ | H | |
| 18 . | N-Oxid von 1 | | | | | Oel |
| 19 . | 5-Br | O | $C_2H_5$ | $C_2H_5$ | H | |
| 20 . | " | O | $n-C_8H_{17}$ | $n-C_8H_{17}$ | H | |
| 21 . | 5,7-$Cl_2$ | O | $C_2H_5$ | $C_2H_5$ | H | 38-40 |
| 22 . | N-Oxid von 21 | | | | | |
| 23 . | 2-$CH_3$ | O | $C_2H_5$ | $C_2H_5$ | H | Oel |
| 24 . | " | O | $C_2H_4OCH_3$ | $C_2H_4OCH_3$ | H | |
| 25 . | 5,7-$Br_2$ | O | $C_2H_5$ | $C_2H_5$ | H | Oel |
| 26 . | 5,7-$Br_2$, 2-$CH_3$ | O | $C_2H_5$ | $C_2H_5$ | H | 70-71 |

C. Biologische Beispiele

Beispiel 1

Weizen und Gerste wurden im Gewächshaus bis zum 3-4 Blattstadium herangezogen und dann

nacheinander mit den erfindungsgemäßen Safenern und einem handelsüblichen Herbizid behandelt. Die Verbindungen wurden in Form wässriger Suspensionen bzw. Emulsionen ausgebracht. Einige Wochen nach der Behandlung (Weizen: 3 Wochen, Gerste: 2 Wochen) wurde das Ausmaß der Pflanzenschädigung durch das Herbizid visuell ermittelt, wobei insbesondere die Wachstumshemmung berücksichtigt wurde. Die Ergebnisse aus Tabelle 2 veranschaulichen, daß die erfindungsgemäßen Verbindungen selbst bei starken Überdosierungen des Herbizids schwere Schädigungen bei den Kulturpflanzen stark reduzieren, geringere Schadsymptome völlig aufheben können. Mischungen aus Herbiziden und erfindungsgemäßen Verbindungen eignen sich deshalb in ausgezeichneter Weise zur selektiven Unkrautbekämpfung in Kulturpflanzen.

Tabelle 2

| Verbindung | Dosis kg/ha | Schädigung in % | |
|---|---|---|---|
| | | TA | HV |
| Herbizid<br>$H_1$ | 2,0<br>0,2 | 85<br>- | -<br>90 |
| 1 + $H_1$ | 2,0 + 2,5<br>0,2 + 2,5 | 25<br>- | -<br>20 |
| 21 + $H_1$ | 2,0 + 2,5<br>0,2 + 2,5 | 40<br>- | -<br>- |
| 18 + $H_1$ | 2,0 + 2,5<br>0,2 + 2,5 | 30<br>- | -<br>40 |
| 7 + $H_1$ | 2,0 + 2,5<br>0,2 + 2,5 | 33<br>- | -<br>75 |
| 16 + $H_1$ | 2,0 + 2,5<br>0,2 + 2,5 | 40<br>- | -<br>75 |
| 5 + $H_1$ | 2,0 + 2,5<br>0,2 + 2,5 | 10<br>- | -<br>30 |
| 13 + $H_1$ | 2,0 + 2,5<br>0,2 + 2,5 | 20<br>- | -<br>50 |
| 14 + $H_1$ | 2,0 + 2,5<br>0,2 + 2,5 | 45<br>- | -<br>75 |
| 12 + $H_1$ | 2,0 + 2,5<br>0,2 + 2,5 | 30<br>- | -<br>40 |
| 11 + $H_1$ | 2,0 + 2,5<br>0,2 + 2,5 | 25<br>- | -<br>60 |
| 23 + $H_1$ | 2,0 + 2,5<br>0,2 + 2,5 | 30<br>- | -<br>- |

Abkürzungen:
$H_1$ = Fenoxaprop-ethyl (Ethyl 2-[4-(6-Chlorbenzoxazolyloxy)phenoxy]-propionat)
TA = Triticum aestivum (Weizen)
HV = Hordeum vulgare (Gerste)

Beispiel 2

Getreide und Schadgräser wurden unter optimalen Wuchsbedingungen im Gewächshaus bis zum 3-4 Blattstadium angezogen und mit Mischungen der erfindungsgemäßen Verbindungen und Herbiziden behandelt. Die Präparate wurden in Form wässriger Suspensionen bzw. Emulsionen ausgebracht. 3-4 Wochen nach Applikation wurden die Versuchspflanzen auf Wachstumsveränderungen und Schädigung im Vergleich zu unbehandelten bzw. mit den Herbiziden allein behandelten Kontrollen visuell bonitiert. Die Ergebnisse

aus Tabelle 3 zeigen, daß die erfindungsgemäßen Verbindungen an Kulturpflanzen wirkungsvoll verhindern können, ohne die herbizide Wirkung gegen Schadgräser zu beeinträchtigen.

Tabelle 3

Safenerwirkung der erfindungsgemäßen Verbindungen bei Weizen und Flughafer

| Verbindung | Dosis kg/ha | herbizide Wirkung in % TA | AVF |
|---|---|---|---|
| $H_1$ | 2,0 | 40 | - |
| | 0,3 | - | 100 |
| $H_1$ + 1 | 2,0 + 1,0 | 15 | - |
| | 2,0 + 0,25 | 10 | - |
| | 0,3 + 1,0 | - | 100 |
| | 0,3 + 0,25 | - | 100 |

Abkürzung: AVF = Avena fatua (Flughafer)

**Patentansprüche**

**Patentansprüche für folgende Vertragsstaaten : BE, CH, DE, FR, GB, IT, LI, NL, SE**

1. Pflanzenschützende Mittel, gekennzeichnet durch einen Gehalt an einer Verbindung der Formel I,

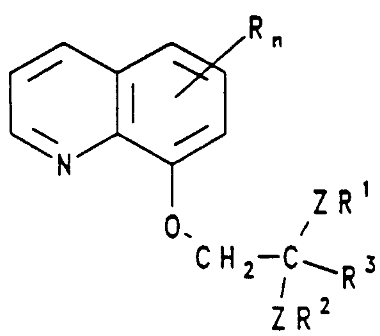

(I)

worin

| | |
|---|---|
| R | Halogen oder $(C_1-C_4)$Alkyl, |
| Z | unabhängig voneinander O oder S, |
| $R^1$, $R^2$ | unabhängig voneinander H, $(C_1-C_8)$Alkyl, $(C_3-C_8)$Alkenyl, $(C_1-C_4)$Alkoxy-$(C_1-C_4)$alkyl, $(C_1-C_{12})$Halogenalkyl mit bis zu 17 Halogenatomen, $(C_3-C_{12})$Halogenalkenyl mit bis zu 17 Halogenatomen oder Phenyl-$(C_1-C_2)$alkyl, |
| $R^3$ | H oder $(C_1-C_4)$Alkyl und |
| n | 0, 1, 2 oder 3, |

bedeuten, oder deren Salzen oder N-Oxiden und an üblichen Formulierungshilfsmitteln.

2. Mittel gemäß Anspruch 1, dadurch gekennzeichnet, daß sie eine Verbindung der Formel I, worin R Halogen, Z Sauerstoff, $R^1$ und $R^2$ $(C_1-C_4)$Alkyl oder $(C_1-C_2)$Alkoxy-$(C_1-C_2)$alkyl, $R^3$ Wasserstoff und n 1 oder 2 bedeuten, enthalten.

3. Mittel gemäß Anspruch 1, dadurch gekennzeichnet, daß sie 5-Chlor-8-chinolyloxy-acetaldehyd-diethylacetal enthalten.

4. Mittel gemäß Anspruch 1, welche zusätzlich ein Herbizid aus der Gruppe der Carbamate, Thiocarbamate, Halogenacetanilide, substituierten Phenoxy-, Naphthoxy, Phenoxy-phenoxycarbonsäurederivate und Heteroaryloxyphenoxycarbonsäurederivate, vorzugsweise Chinolyloxy, Chinoxalyloxy, Pyridyloxy, Benzoxazolyloxy- und Benzthiazolyloxyphenoxycarbonsäurederivate, und Dimedonoximabkömmlinge enthalten.

5. Mittel gemäß Anspruch 4, dadurch gekennzeichnet, daß sie als Herbizid Fenoxaprop-ethyl enthalten.

6. Verfahren zum Schutz von Kulturpflanzen gegen phytotoxische Nebenwirkungen von Herbiziden, bei welchem man die Pflanzen, Pflanzenteile oder Pflanzennährböden vor, nach oder gleichzeitig mit der Anwendung eines Herbizids mit einer Verbindung gemaß Anspruch 1 (I) in subtoxischer Konzentration mit einem Mengenverhältnis (I) : Herbizid zwischen 0,01:1 und 10:1 behandelt.

7. Verfahren gemäß Anspruch 6, bei welchem man eine Verbindung gemäß Anspruch 1 in Kombination mit einem in Anspruch 4 definierten Herbizid anwendet.

8. Verfahren gemäß Anspruch 6, dadurch gekennzeichnet, daß man die Verbindungen in Kombination mit Fenoxaprop-ethyl anwendet.

9. Verwendung von Verbindungen gemäß Anspruch 1 zum Schutz von Kulturpflanzen gegen phytotoxische Nebenwirkungen von Herbiziden.

10. Verbindungen der Formel I gemäß Anspruch 1 mit Ausnahme von 8-Chinolyloxy-acetaldehyd-diethylacetal.

11. Verbindungen der Formel I gemäß Anspruch 1, worin R Halogen, Z Sauerstoff, $R^1$ und $R^2$ $(C_1-C_4)$Alkyl oder $(C_1-C_2)$Alkoxy-$(C_1-C_2)$alkyl, $R^3$ Wasserstoff und n 1 oder 2 bedeuten.

12. Verfahren zur Herstellung von Verbindungen gemäß Anspruch 10, dadurch gekennzeichnet, daß man Verbindungen der Formel II

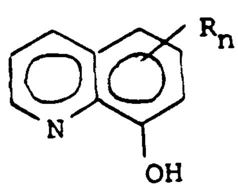

(II)

mit Verbindungen der Formel (III)

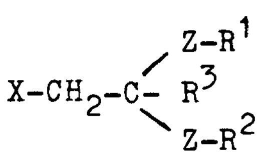

(III),

worin die Reste die oben angegebene Bedeutung haben und X für eine Abgangsgruppe steht, umgesetzt, und gewünschtenfalls die so erhaltene Acetale der Formel (I) durch Umacetalisierung in andere Derivate der Formel (I) bzw. Verbindungen der Formel I in ihre Salze oder N-Oxide überführt.

**Patentansprüche für folgenden Vertragsstaat : AT**

**1.** Pflanzenschützende Mittel, gekennzeichnet durch einen Gehalt an einer Verbindung der Formel I,

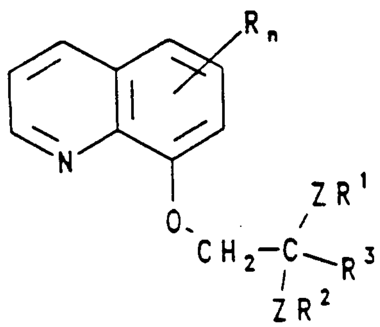

(I)

worin

| | |
|---|---|
| R | Halogen oder $(C_1\text{-}C_4)$Alkyl, |
| Z | unabhängig voneinander O oder S, |
| $R^1$, $R^2$ | unabhängig voneinander H, $(C_1\text{-}C_8)$Alkyl, $(C_3\text{-}C_8)$Alkenyl, $(C_1\text{-}C_4)$Alkoxy-$(C_1\text{-}C_4)$alkyl, $(C_1\text{-}C_{12})$Halogenalkyl mit bis zu 17 Halogenatomen, $(C_3\text{-}C_{12})$Halogenalkenyl mit bis zu 17 Halogenatomen oder Phenyl-$(C_1\text{-}C_2)$alkyl, |
| $R^3$ | H oder $(C_1\text{-}C_4)$Alkyl und |
| n | 0, 1, 2 oder 3 |

bedeuten, oder deren Salzen oder N-Oxiden und an üblichen Formulierungshilfsmitteln.

**2.** Mittel gemäß Anspruch 1, dadurch gekennzeichnet, daß sie eine Verbindung der Formel I, worin R Halogen, Z Sauerstoff, $R^1$ und $R^2$ $(C_1\text{-}C_4)$Alkyl oder $(C_1\text{-}C_2)$Alkoxy-$(C_1\text{-}C_2)$alkyl, $R^3$ Wasserstoff und n 1 oder 2 bedeuten, enthalten.

**3.** Mittel gemäß Anspruch 1, dadurch gekennzeichnet, daß sie 5-Chlor-8-chinolyloxy-acetaldehyd-diethylacetal enthalten.

**4.** Mittel gemäß Anspruch 1, welche zusätzlich ein Herbizid aus der Gruppe der Carbamate, Thiocarbamate, Halogenacetanilide, substituierten Phenoxy-, Naphthoxy-, Phenoxy-phenoxycarbonsäurederivate und Heteroaryloxyphenoxycarbonsäurederivate, vorzugsweise Chinolyloxy-, Chinoxalyloxy-, Pyridyloxy-, Benzoxazolyloxy- und Benzthiazolyloxy-phenoxycarbonsäurederivate, und Dimedonoximabkömmlinge enthalten.

**5.** Mittel gemäß Anspruch 4, dadurch gekennzeichnet, daß sie als Herbizid Fenoxaprop-ethyl enthalten.

**6.** Verfahren zum Schutz von Kulturpflanzen gegen phytotoxische Nebenwirkungen von Herbiziden, bei welchem man die Pflanzen, Pflanzenteile oder Pflanzennährböden vor, nach oder gleichzeitig mit der Anwendung eines Herbizids mit einer Verbindung gemäß Anspruch 1 (I) in subtoxischer Konzentration mit einem Mengenverhältnis (I): Herbizid zwischen 0,01:1 und 10:1 behandelt.

**7.** Verfahren gemäß Anspruch 6, bei welchem man eine Verbindung gemäß Anspruch 1 in Kombination mit einem in Anspruch 4 definierten Herbizid anwendet.

**8.** Verfahren gemäß Anspruch 6, dadurch gekennzeichnet, daß man die Verbindungen in Kombination mit Fenoxaprop-ethyl anwendet.

**9.** Verwendung von Verbindungen gemäß Anspruch 1 zum Schutz von Kulturpflanzen gegen phytotoxische Nebenwirkungen von Herbiziden.

**Patentansprüche für folgenden Vertragsstaat : ES**

**1.** Verfahren zur Herstellung von Verbindungen der Formel I

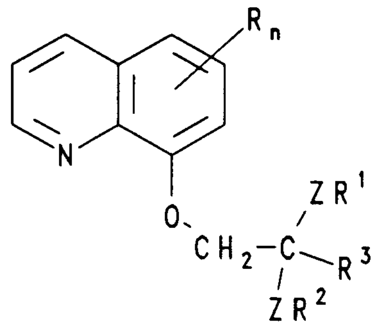

(I)

in welcher die einzelnen Reste folgende Bedeutung haben:

| | |
|---|---|
| R | Halogen oder $(C_1-C_4)$Alkyl, |
| Z | unabhängig voneinander O oder S, |
| $R^1$, $R^2$ | unabhängig voneinander H, $(C_1-C_8)$Alkyl, $(C_3-C_8)$Alkenyl, $(C_1-C_4)$Alkoxy-$(C_1-C_4)$alkyl, $(C_1-C_{12})$Halogenalkyl mit bis zu 17 Halogenatomen, $(C_3-C_{12})$Halogenalkenyl mit bis zu 17 Halogenatomen oder Phenyl-$(C_1-C_2)$alkyl, |
| $R^3$ | H oder $(C_1-C_4)$Alkyl, |
| n | 0, 1, 2 oder 3 |

bzw. deren Salzen und N-Oxiden mit Ausnahme des 8-Chinolyloxyacetaldehyd-diethylacetals, dadurch gekennzeichnet, daß man Verbindungen der Formel II

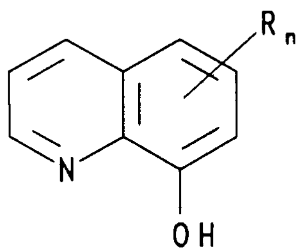

(II)

mit Verbindungen der Formel III

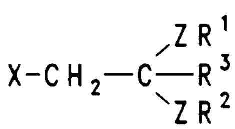

(III)

worin die Reste die oben angegebene Bedeutung haben und X für eine Abgangsgruppe steht, umgesetzt, und gewünschtenfalls die so erhaltenen Acetale der Formel (I) durch Umacetalisierung in andere Derivate der Formel (I) bzw. Verbindungen der Formel I in ihre Salze oder N-Oxide überführt.

**2.** Verfahren gemäß Anspruch 1, dadurch gekennzeichnet, daß in der Formel I gemäß Anspruch 1 R Halogen, Z Sauerstoff, $R^1$ und $R^2$ $(C_1-C_4)$Alkyl oder $(C_1-C_2)$Alkoxy-$(C_1-C_2)$alkyl, $R^3$ Wasserstoff und n 1 oder 2 bedeuten.

**3.** Verfahren gemäß Anspruch 1, dadurch gekennzeichnet, daß die Verbindung 5-Chlor-8-chinolyloxy-acetaldehyd-diethylacetal ist.

**Claims**

**Claims for the following Contracting States : BE, CH, DE, FR, GB, IT, LI, NL, SE**

**1.** A crop protection agent which contains a compound of the formula I

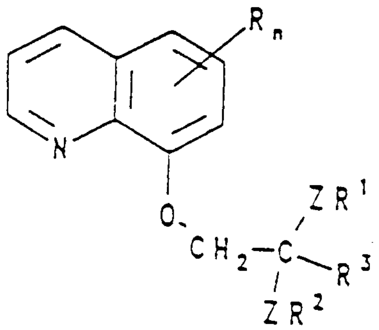

(I)

in which

R denotes halogen or ($C_1$-$C_4$) alkyl,

Z, in each case independently of one another, denotes O or S,

$R^1$ and $R^2$, independently of one another, denote H, ($C_1$-$C_8$)alkyl, ($C_3$-$C_8$)alkenyl, ($C_1$-$C_4$)alkoxy-($C_1$-$C_4$)alkyl, ($C_1$-$C_{12}$)haloalkyl having up to 17 halogen atoms, ($C_3$-$C_{12}$)haloalkenyl having up to 17 halogen atoms or phenyl-($C_1$-$C_2$)alkyl,

$R^3$ denotes H or ($C_1$-$C_4$)alkyl, and

n denotes 0, 1, 2 or 3

or the salts or N-oxides thereof, and contains customary formulation auxiliaries.

**2.** An agent as claim in claim 1, which contains a compound of the formula I, in which R denotes halogen Z denotes oxygen, $R^1$ and $R^2$ denote ($C_1$-$C_4$)alkyl or ($C_1$-$C_2$)alkoxy-($C_1$-$C_2$)alkyl, $R^3$ denotes hydrogen and n denotes 1 or 2.

**3.** An agent, as claimed in claim 1, which contains 5-chloro-8-quinolyloxy-acetaldehyde diethyl acetal.

**4.** An agent as claimed in claim 1, which contains, in addition, a herbicide from the group comprising the carbamates, thiocarbamates, haloacetanilides, substituted phenoxy-, naphthoxy-, phenoxyphenoxy- and heteroaryloxyphenoxy-carboxylic acid derivatives, preferably quinolyloxy-, quinoxalyloxy-, pyridyloxy-, benzoxazolyloxy- and benzothiazolyloxyphenoxy-carboxylic acid derivatives, and dimedone oxime derivatives.

**5.** An agent as claimed in claim 4, wherein the herbicide is fenoxaprop-ethyl.

**6.** A process for protecting crop plants against phytotoxic side effects of herbicides, wherein the plants, parts of plants or nutritive substrata of plants are treated with a compound as claimed in claim 1, in a subtoxic concentration with a ratio of (I) to herbicide of between 0.01:1 and 10:1, before, after or simultaneously with the application of a herbicide.

**7.** The process as claimed in claim 6, wherein a compound as claimed in claim 1 is applied in combination with a herbicide as defined in claim 4.

**8.** The process as claimed in claim 6, wherein the compounds are applied in combination with fenoxaprop-ethyl.

**9.** The use of a compound as claimed in claim 1 for protecting crop plants against phytotoxic side effects of herbicides.

**10.** A compound of the formula I as defined in claim 1, with the exception of 8-quinolyloxy-acetaldehyde diethyl acetal.

**11.** A compound of the formula I as defined in claim 1, in which R denotes halogen, Z denotes oxygen, $R^1$ and $R^2$ denote ($C_1$-$C_4$)alkyl or ($C_1$-$C_2$)alkoxy-($C_1$-$C_2$)alkyl, $R^3$ denotes hydrogen and n denotes 1 or 2.

**12.** A process for the preparation of a compound as claimed in claim 10, wherein compounds of the formula II

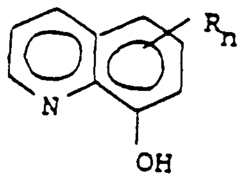

(II)

are reacted with compounds of the formula (III)

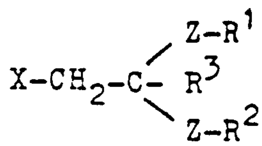

(III),

in which the radicals have the above-mentioned meaning and X represents a leaving group, and, if desired, the acetals of the formula (I) thus obtained are converted, by transacetalization, into other derivatives of the formula (I) or compounds of the formula I are converted into their salts or N-oxides.

**Claims for the following Contracting State : AT**

**1.** A crop protection agent which contains a compound of the formula I

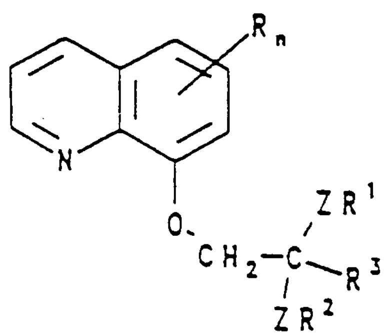

(I)

in which

| | |
|---|---|
| R | denotes halogen or ($C_1$-$C_4$) alkyl, |
| Z, | in each case independently of one another, denotes O or S, |
| $R^1$ and $R^2$, | independently of one another, denote H, ($C_1$-$C_8$)alkyl, ($C_3$-$C_8$)alkenyl, ($C_1$-$C_4$)alkoxy-($C_1$-$C_4$)alkyl, ($C_1$-$C_{12}$)haloalkyl having up to 17 halogen atoms, ($C_3$-$C_{12}$)haloalkenyl having up to 17 halogen atoms or phenyl-($C_1$-$C_2$)alkyl, |
| $R^3$ | denotes H or ($C_1$-$C_4$)alkyl, and |
| n | denotes 0, 1, 2 or 3 |
| | or the salts or N-oxides thereof, and contains customary formulation auxiliaries. |

**2.** An agent as claim in claim 1, which contains a compound of the formula I, in which R denotes halogen Z denotes oxygen, $R^1$ and $R^2$ denote ($C_1$-$C_4$)alkyl or ($C_1$-$C_2$)alkoxy-($C_1$-$C_2$)alkyl, $R^3$ denotes hydrogen and n denotes 1 or 2.

**3.** An agent, as claimed in claim 1, which contains 5-chloro-8-quinolyloxy-acetaldehyde diethyl acetal.

**4.** An agent as claimed in claim 1, which contains, in addition, a herbicide from the group comprising the

carbamates, thiocarbamates, haloacetanilides, substituted phenoxy-, naphthoxy-, phenoxyphenoxy- and heteroaryloxyphenoxy-carboxylic acid derivatives, preferably quinolyloxy-, quinoxalyloxy-, pyridyloxy-, benzoxazolyloxy- and benzothiazolyloxy-phenoxy-carboxylic acid derivatives, and dimedone oxime derivatives.

**5.** An agent as claimed in claim 4, wherein the herbicide is fenoxaprop-ethyl.

**6.** A process for protecting crop plants against phytotoxic side effects of herbicides, wherein the plants, parts of plants or nutritive substrata of plants are treated with a compound as claimed in claim 1, in a subtoxic concentration with a ratio of (I) to herbicide of between 0.01:1 and 10:1, before, after or simultaneously with the application of a herbicide.

**7.** The process as claimed in claim 6, wherein a compound as claimed in claim 1 is applied in combination with a herbicide as defined in claim 4.

**8.** The process as claimed in claim 6, wherein the compounds are applied in combination with fenoxaprop-ethyl.

**9.** The use of a compound as claimed in claim 1 for protecting crop plants against phytotoxic side effects of herbicides.

**Claims for the following Contracting State : ES**

**1.** A process for the preparation of a compound of the formula I

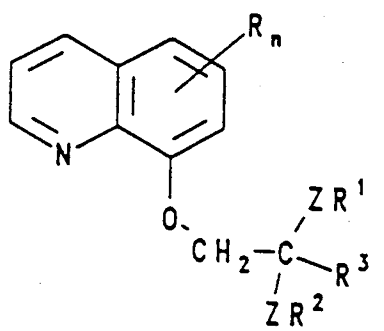

(I)

in which the individual radicals have the following meaning:

| | |
|---|---|
| R | denotes halogen or $(C_1-C_4)$alkyl, |
| Z, | in each case independently of one another, denotes O or S, |
| $R^1$ and $R^2$, | independently of one another, denote H, $(C_1-C_8)$alkyl, $(C_3-C_8)$alkenyl, $(C_1-C_4)$alkoxy-$(C_1-C_4)$alkyl, $(C_1-C_{12})$haloalkyl having up to 17 halogen atoms, $(C_3-C_{12})$haloalkenyl having up to 17 halogen atoms or phenyl-$(C_1-C_2)$alkyl, |
| $R^3$ | denotes H or $(C_1-C_4)$alkyl, and |
| n | denotes 0, 1, 2 or 3 |

and the salts and N-oxides thereof, with the exception of 8-quinolyloxy-acetaldehyde diethyl acetal which comprises reacting compounds of the formula II

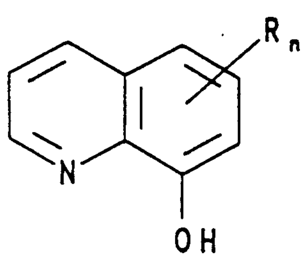

(II)

with compounds of the formula III

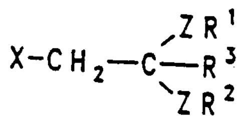

(III),

in which the radicals have the abovementioned meaning and X represents a leaving group, and, if desired, the acetals of the formula (I) thus obtained are converted, by transacetalization, into other derivatives of the formula (I) or converting compounds of the formula I into their salts or N-oxides.

**2.** The process as claimed in claim 1, wherein in the formula I as claimed in claim 1 R denotes halogen, Z denotes oxygen, $R^1$ and $R^2$ denote $(C_1\text{-}C_4)$alkyl or $(C_1\text{-}C_2)$alkoxy-$(C_1\text{-}C_2)$alkyl, $R^3$ denotes hydrogen and n denotes 1 or 2.

**3.** The process as claimed in claim 1, wherein the compound is 5-chloro-8-quinolyloxy-acetaldehyde diethyl acetal.

**Revendications**
**Revendications pour les Etats contractants suivants : BE, CH, DE, FR, GB, IT, LI, NL, SE**

**1.** Agents de protection des plantes (produits phytosanitaires), caractérisés par une teneur en un composé de formule I :

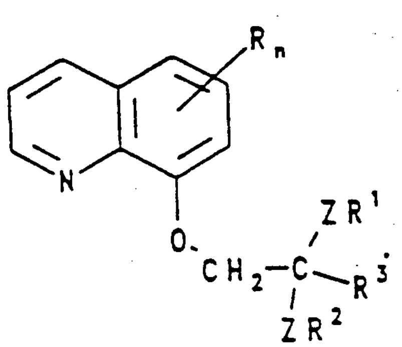

(I)

dans laquelle :

| | |
|---|---|
| R | représente un atome d'halogène ou un groupe alkyle en $C_1$ à $C_4$, |
| les Z | représentent chacun, indépendamment l'un de l'autre, O ou S, |
| $R^1$ et $R^2$ | représentent chacun, indépendamment l'un de l'autre H, un groupe alkyle en $C_1$ à $C_8$, alcényle en $C_3$ à $C_8$, alcoxy (en $C_1$ à $C_4$)-alkyle (en $C_1$ à $C_4$), halogénoalkyle en $C_1$ à $C_{12}$ comportant jusqu'à 17 atomes d'halogène, halogénoalcényle en $C_3$ à $C_{12}$ comportant jusqu'à 17 atomes d'halogène ou phényl-alkyle en $C_1$ ou $C_2$, |
| $R^3$ | représente H ou un groupe alkyle en $C_1$ à $C_4$, et |
| n | est-un nombre valant 0, 1, 2 ou 3, |

ou leurs sels ou leurs N-oxydes et des adjuvants usuels de formulation.

**2.** Produits selon la revendication 1, caractérisés en ce qu'ils contiennent un composé de formule I, dans laquelle R représente un atome d'halogène, Z représente un atome d'oxygène, $R^1$ et $R^2$ représentent chacun un groupe alkyle en $C_1$ à $C_4$ ou alcoxy (en $C_1$ ou $C_2$)-alkyle (en $C_1$ ou $C_2$) ; $R^2$ représente un atome d'hydrogène et n vaut 1 ou 2.

**3.** Produits selon la revendication 1, caractérisés en ce qu'ils contiennent du diéthylacétal de 5-chloro-8-quinonyloxy-acétaldéhyde.

**4.** Produits selon la revendication 1 qui peuvent contenir en outre un herbicide choisi dans l'ensemble

formé par des carbamates, des thiocarbamates, des halogénoacétanilides, des dérivés substitués d'acides phénoxy-, naphtoxy-, phénoxy-phénoxycarboxyliques et des dérivés d'acides hétéroaryloxyphénoxycarboxyliques, avantageusement des dérivés d'un acide quinonyloxy-, quinoxalyloxy-, pyridyloxy-, benzoxazolyloxy- et benzothiazolyloxy-phénoxycarboxyliques, et des dérivés de dimédonoximes.

**5.** Produits selon la revendication 4, caractérisés en ce qu'ils contiennent comme herbicide du fénoxaprop-éthyl.

**6.** Procédé pour protéger des plantes cultivées contre les effets phytotoxiques secondaires d'herbicides, selon lequel on traite les plantes, des parties de plantes ou des sols destinés à nourrir des plantes avant, après ou en même temps que l'utilisation d'un herbicide, avec un composé selon la revendication 1 (I) en une concentration subtoxique et selon un rapport compris entre 0,01:1 et 10:1 entre les quantités du composé (1) et de l'herbicide.

**7.** Procédé selon la revendication 6, dans lequel on applique un composé selon la revendication 1 en combinaison avec un herbicide défini à la revendication 4.

**8.** Procédé selon la revendication 6, caractérisé en ce qu'on applique les composés en combinaison avec du fénoxaprop-éthyl.

**9.** Utilisation de composés selon la revendication 1 pour protéger des plantes cultivées contre les effets phytotoxiques secondaires d'herbicides.

**10.** Composés de formule I selon la revendication 1, à l'exclusion du diéthylacétal de 8-quinonyloxy-acétaldéhyde.

**11.** Composés de formule I selon la revendication 1, dans laquelle R représente un atome d'halogène, Z représente un atome d'oxygène, $R^1$ et $R^2$ représentent chacun un groupe alkyle en $C_1$ à $C_4$ ou alcoxy (en $C_1$ ou $C_2$)-alkyle (en $C_1$ ou $C_2$), $R^3$ représente un atome d'hydrogène et n vaut 1 ou 2.

**12.** Procédé pour préparer des composés selon la revendication 10, caractérisé en ce qu'on fait réagir des composés de formule II :

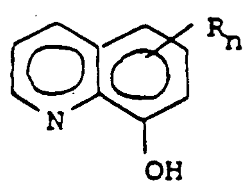

(II)

avec des composés de formule III :

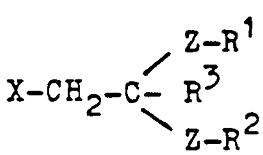

(III),

dans lesquels les restes ont le sens indiqué ci-dessus et X représente un groupe partant, et l'on transforme éventuellement les acétals ainsi obtenus, de formule (I) par transacétalisation en d'autres dérivés de formule (I) ou bien l'on transforme des composés de formule I en leurs sels ou en leurs N-oxydes.

**Revendications pour l'Etat contractant suivant : AT**

1. Produits phytosanitaires pour la protection des plantes, caractérisés en ce qu'ils contiennent un composé de formule I :

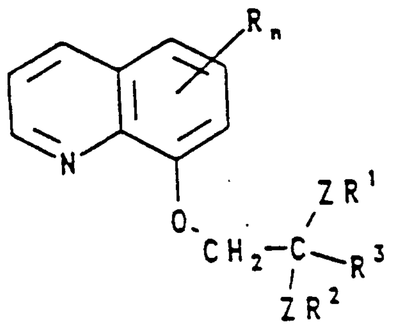

(I)

dans laquelle :

| | |
|---|---|
| les symboles R | représentent chacun un atome d'halogène ou un groupe alkyle en $C_1$ à $C_4$, |
| les Z | représentent chacun, indépendamment l'un de l'autre, O ou S, |
| $R^1$ et $R^2$ | représentent chacun, indépendamment l'un de l'autre, H, un groupe alkyle en $C_1$ à $C_8$, alcényle en $C_3$ à $C_8$, alcoxy (en $C_1$ à $C_4$) alkyle (en $C_1$ à $C_4$), halogénoalkyle en $C_1$ à $C_{12}$ comportant jusqu'à 17 atomes d'halogène, halogénoalcényle en $C_3$ à $C_{12}$ comportant jusqu'à 17 atomes d'halogène ou un groupe phényle-alkyle (en $C_1$ ou $C_2$), |
| $R^3$ | représente H ou un groupe alkyle en $C_1$ à $C_4$, et |
| n | est un nombre valant 0, 1, 2 ou 3, |

ou leurs sels ou leurs N-oxydes et un adjuvant usuel de formulation.

2. Produits selon la revendication 1, caractérisés en ce qu'ils contiennent un composé de formule I, dans laquelle R représente un atome d'halogène, Z représente un atome d'oxygène, $R^1$ et $R^2$ représentent chacun un groupe alkyle en $C_1$ à $C_4$ ou alcoxy (en $C_1$ ou $C_2$)-alkyle (en $C_1$ ou $C_2$) ; $R^2$ représente un atome d'hydrogène et n vaut 1 ou 2.

3. Produits selon la revendication 1, caractérisés en ce qu'ils contiennent du diéthylacétal de 5-chloro-8-quinonyloxy-acétaldéhyde.

4. Produits selon la revendication 1 qui peuvent contenir en outre un herbicide choisi dans l'ensemble formé par des carbamates, des thiocarbamates, des halogénoacéta ilides, des dérivés substitués d'acides phénoxy, naphtoxy-, phénoxy-phénoxycarboxyliques et des dérivés d'acides hétéroaryloxyphénoxycarboxyliques, avantageusement des dérivés d'un acide quinonyloxy-, quinoxalyloxy-, pyridyloxy-, benzoxazolyloxy- et benzothiazolyloxy-phénoxycarboxyliques, et des dérivés de dimédonoximes.

5. Produits selon la revendication 4, caractérisés en ce qu'ils contiennent comme herbicide du fénoxaprop-éthyl.

6. Procédé pour protéger des plantes cultivées contre les effets phytotoxiques secondaires d'herbicides, selon lequel on traite les plantes, des parties de plantes ou des sols destinés à nourrir des plantes avant, après ou en même temps que l'utilisation d'un herbicide, avec un composé selon la revendication 1 (I) en une concentration subtoxique et selon un rapport compris entre 0,01:1 et 10:1 entre les quantités du composé (1) et de l'herbicide.

7. Procédé selon la revendication 5, dans lequel on applique un composé selon la revendication 1 en combinaison avec un herbicide défini à la revendication 4.

8. Procédé selon la revendication 5, caractérisé en ce qu'on applique les composés en combinaison avec du fénoxaprop-éthyl.

9. Utilisation de composés selon la revendication 1 pour protéger des plantes cultivées contre les effets phytotoxiques secondaires d'herbicides.

**Revendications pour l'Etat contractant suivant : ES**

1. Procédé pour préparer des composés de formule I :

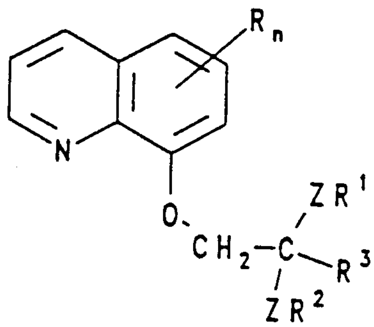

(I)

dans laquelle les divers restes ont les sens suivants :

les R représentent chacun un atome d'halogène ou un groupe alkyle en $C_1$ à $C_4$,

les Z représentent chacun, indépendamment l'un de l'autre, O ou S,

$R^1$ et $R^2$ représentent chacun, indépendamment l'un de l'autre, H, un groupe alkyle en $C_1$ à $C_8$, alcényle en $C_3$ à $C_8$, alcoxy (en $C_1$ à $C_4$) alkyle (en $C_1$ à $C_4$), halogénoalkyle en $C_1$ à $C_{12}$ comportant jusqu'à 17 atomes d'halogène, halogénoalcényle en $C_3$ à $C_{12}$ comportant jusqu'à 17 atomes d'halogène ou phényl-alkyle en $C_1$ ou $C_2$,

$R^3$ représente H ou un groupe alkyle en $C_1$ à $C_4$,

n vaut 0, 1, 2 ou 3,

ou leurs sels et leurs N-oxydes, à l'exclusion du diéthylacétal de 8-quinolyloxyacétaldéhyde, procédé caractérisé en ce qu'on fait réagir des composés de formule II :

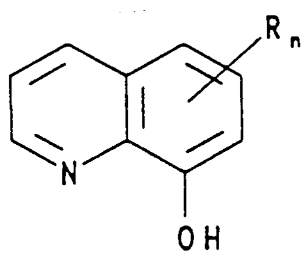

(II)

avec des composés de formule III :

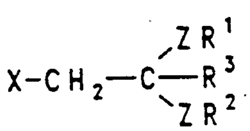

(III)

dans lesquelles les restes ont les sens indiqués ci-dessus et X représente un groupe partant et, éventuellement, on transforme les acétals ainsi obtenus, de formule (I), par transacétalisation en d'autres dérivés de formule (I) ou bien on transforme les composés de formule I en leurs sels ou en leurs N-oxydes.

2. Procédé selon la revendication 1, caractérisé en ce que, dans la formule I selon la revendication 1, R représente un atome d'halogène, Z représente un atome d'oxygène, $R^1$ et $R^2$ représentent chacun un groupe alkyle en $C_1$ à $C_4$ ou alcoxy (en $C_1$ ou $C_2$)-alkyle (en $C_1$ ou $C_2$), $R^3$ représente un atome

d'hydrogène et n vaut 1 ou 2.

**3.** Procédé selon la revendication 1, caractérisé en ce que le composé est le diéthylacétal de 5-chloro-8-quinonyloxy-acétaldéhyde.